# EUROPÄISCHE PATENTSCHRIFT

(11) **EP 0 491 226 B1**
(45) Veröffentlichungstag und Bekanntmachung des Hinweises auf die Patenterteilung: **14.08.1996**
(21) Anmeldenummer: 91120745.4
(22) Anmeldetag: 03.12.1991
(51) Int. Cl.: C07D 213/55, A61K 31/44

(54) **Substituierte Pyridyl-dihydroxy-heptensäure und deren Salze**
Substituted pyridyl-dihydroxyheptenoic acid and its salts
Acide pyridyl dihydroxy hepténoique substitué et ses sels

(30) Priorität: 14.12.1990 DE 4040026; 31.07.1991 IT MI912125
(43) Veröffentlichungstag der Anmeldung: 24.06.1992
(73) Patentinhaber: BAYER AG, 51368 Leverkusen (DE)
(72) Erfinder: Angerbauer, Rolf, Dr., W-5600 Wuppertal (DE); Fey, Peter, Dr., W-5600 Wuppertal (DE); Hübsch, Walter, Dr., W-5600 Wuppertal (DE); Philipps, Thomas, Dr., W-5000 Köln 80 (DE); Bischoff, Hilmar, Dr., W-5600 Wuppertal (DE); Petzinna, Dieter, Dr., W-4000 Düsseldorf 12 (DE); Schmidt, Delf, Dr., W-5600 Wuppertal (DE); Thomas, Günter, Dr., W-5600 Wuppertal (DE)

(56) Entgegenhaltungen:
- EP-A- 0 306 929
- EP-A- 0 307 342
- EP-A- 0 325 130
- EP-A- 0 356 788

## Beschreibung

Die Erfindung betrifft eine substituierte Pyridyl-dihydroxy-heptensäure, deren Salze, Verfahren zur Herstellung und die Verwendung in Arzneimitteln.

Es ist bekannt, daß aus Pilzkulturen isolierte Lacton-Derivate Inibitoren der 3-Hydroxy-3-methyl-glutaryl Coenzym A Reduktase (HMG-CoA-Reduktase) sind [Mevinolin, EP 22 478; US-4 231 938].

Außerdem ist bekannt, daß Pyridin-substituierte Dihydroxyheptensäuren Inhibitoren der HMG-CoA-Reduktase sind [EP 325 130; EP 356 788 EP 307 342; EP 306 929].

Es wurde nun gefunden, daß die substituierte Pyridyl-dihydroxy-heptensäure der Formel 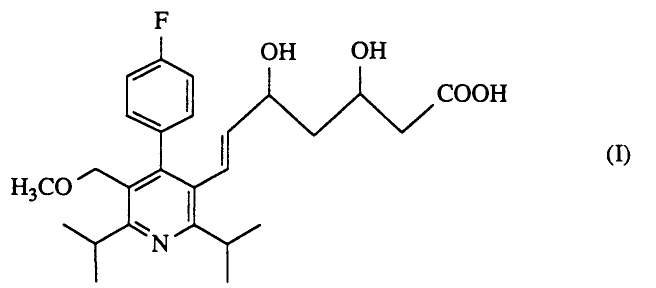 und deren Salze, gegebenenfalls in einer isomeren Form, eine überlegene inhibitorische Wirkung auf die HMG-CoA-Reduktase aufweisen und somit eine überraschend gute Senkung des Cholesteringehalts im Blut bewirken.

Die erfindungsgemäße substituierte Pyridyl-dihydroxy-heptensäure kann in Form ihrer Salze vorliegen. Im allgemeinen seien hier Salze mit organischen oder anorganischen Basen genannt.

Im Rahmen der vorliegenden Erfindung werden physiologisch unbedenkliche Salze bevorzugt. Physiologisch unbedenkliche Salze der erfindungsgemäßen substituierten Pyridyl-dihydroxy-heptensäure können Metall- oder Ammoniumsalze sein. Bevorzugt zu nennen seien Natrium-, Kalium-, Magnesium- oder Calciumsalze sowie Ammoniumsalze, die abgeleitet sind von Ammoniak oder organischen Aminen wie beispielsweise Methylamin, Ethylamin, Propylamin, Isopropylamin, Di- bzw. Triethylamin, Diisopropylamin, Di- bzw. Triethanolamin, Dicyclohexylamin, Arginin, Lysin oder Ethylendiamin. Besonders bevorzugt sind Natrium- und Kaliumsalze.

Die erfindungsgemäße substituierte Pyridyl-dihydroxy-heptensäure sowie deren Salze haben zwei asymmetrische Kohlenstoffatome, nämlich die beiden Kohlenstoffatome, an denen die Hydroxygruppen gebunden sind, und können daher in verschiedenen stereochemischen Formen existieren. Die Erfindung betrifft sowohl die einzelnen Isomeren als auch deren Mischungen. So können die erfindungsgemäßen Stoffe je nach der relativen Stellung der Hydroxygruppen in der Erythro-Konfiguration oder in der Threo-Konfiguration vorliegen:

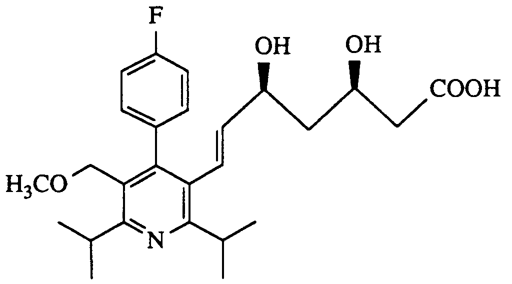

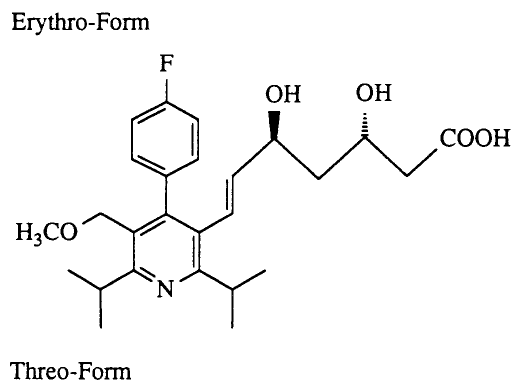

Die Erythro-Konfiguration ist bevorzugt.

Sowohl von den Stoffen in Threo- als auch in Erythro-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form). Bevorzugt sind hiervon die 3R,5S/3S,5R-Racemate sowie die 3R,5S-Enantiomere.

Darüber hinaus können die erfindungsgemäßen Stoffe aufgrund der Doppelbindung in E-Konfiguration oder der Z-Konfiguration vorliegen. Bevorzugt sind diejenigen Verbindungen, die E-konfiguriert sind.

Besonders bevorzugt sind die (+)-Enantiomere der substituierten Pyridyl-dihydroxy-heptensäure in der Erythro-(E)-Konfiguration sowie deren Salze.

Die substituierte Pyridyl-dihydroxy-heptensäure der Formel (I) 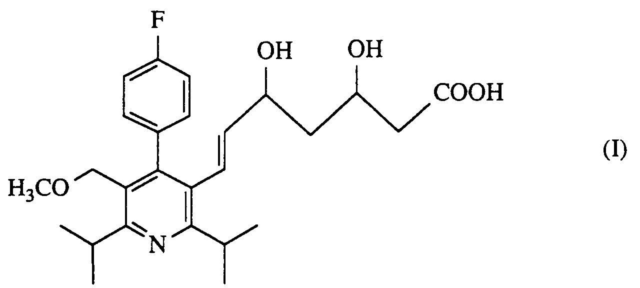 sowie deren Salze, gegebenenfalls in einer isomeren Form,
werden hergestellt, indem man
[A] im Fall der racemischen Produkte die entsprechenden racemischen Ester der Formel (II) 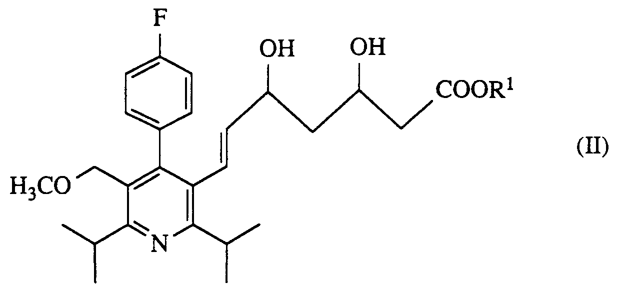 in welcher
   - R¹ -: für C₁-C₄-Alkyl oder Benzyl steht,
   hydrolysiert, oder
[B] im Fall der stereoisomer einheitlichen Produkte
   zunächst die racemischen Ester der Formel (II)
   mit dem(+)- oder (-)-Enantiomeren Amin der Formel (III) 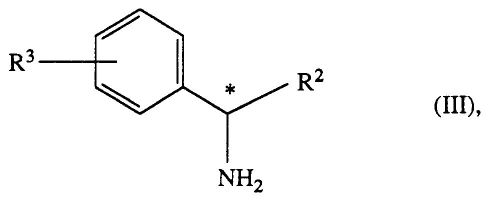 in welcher
   - R²-: für gegebenenfalls durch Hydroxy substituiertes C₁-C₄-Alkyl steht
   und
   - R³ -: für Wasserstoff, Halogen, C₁-C₄-Alkyl oder C₁-C₄-Alkoxy steht,
   in die entsprechenden diastereomeren Amide der Formel (IV) 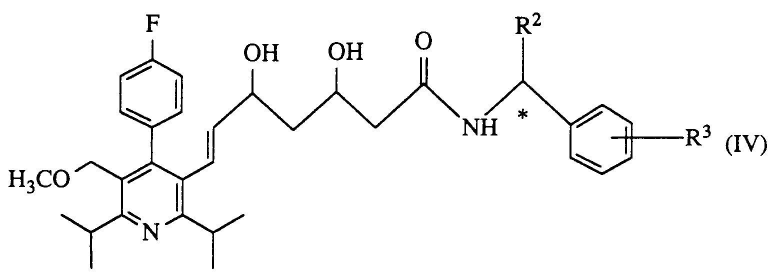 überführt,
das Gemisch der diastereomeren Amide durch Chromatographie oder Kristallisation in die einzelnen Diastereomeren trennt,
und anschließend die reinen diastereomeren Amide zu den enantiomerenreinen Produkten hydrolysiert.

Das Verfahren soll im folgenden Schema beispielhaft erläutert werden:

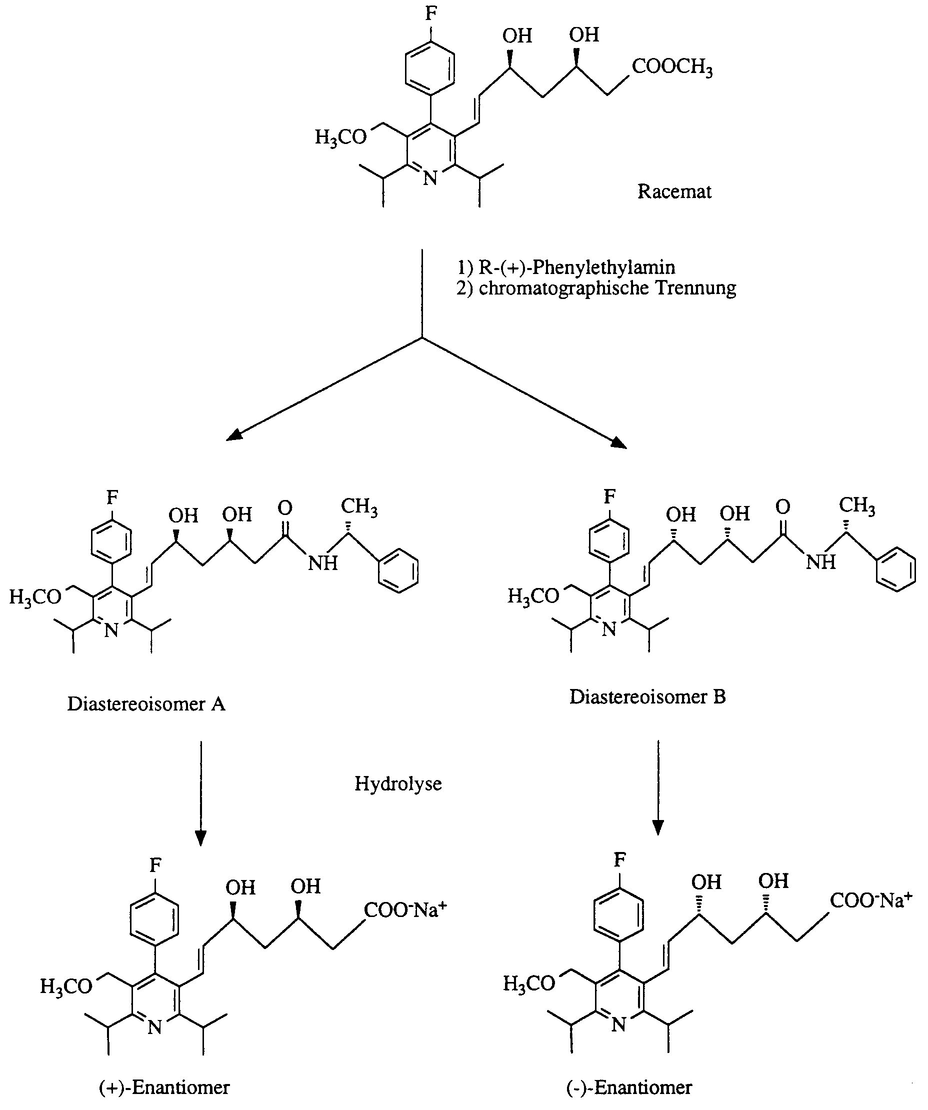

Die Hydrolyse der Ester (II) erfolgt im allgemeinen, indem man die Ester in inerten Lösemitteln mit Basen behandelt, wobei im allgemeinen zunächst die Salze entstehen, die anschließend in einem zweiten Schritt durch Behandeln mit Säure in die freie Säure (I) überführtwerden.

Als Lösemittel eignen sich für die Hydrolyse der Ester Wasser oder die für eine Verseifung von Estern üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Als Basen eignen sich für die Hydrolyse der Ester die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.-butanolat. Besonders bevorzugt werden Natriumhydroxid oder Kaliumhydroxid eingesetzt.

Die Hydrolyse der Ester wird im allgemeinen in einem Temperaturbereich von -10°C bis +100°C, bevorzugt von +20°C bis +80°C durchgeführt.

Im allgemeinen wird die Hydrolyse der Ester bei Normaldruck durchgeführt. Es ist aber auch möglich, bei Unterdruck oder bei Überdruck zu arbeiten (z.B. von 0,5 bis 5 bar).

Bei der Durchführung der Hydrolyse wird die Base im allgemeinen in einer Menge von 1 bis 3 Mol, bevorzugt von 1 bis 5 Mol, bezogen auf 1 Mol des Esters eingesetzt. Besonders bevorzugt verwendet man molare Mengen an Reaktanden.

Bei der Durchführung der Hydrolyse entstehen im ersten Schritt die Salze der erfindungsgemäßen Säure, die isoliert werden können. Die erfindungsgemäße Säure erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäure hierbei als vorteilhaft erwiesen, die basische Reaktionsmischung der Hydrolyse in einem zweiten Schritt ohne Isolierung der Salze anzusäuern. Die Säure kann dann in üblicher Weise isoliert werden.

Die Umsetzung der Ester (II) mit den enantiomerenreinen Aminen (III) zu den diastereomeren Amiden (IV) erfolgt im allgemeinen in inerten Lösemitteln.

Als Lösemittel eignen sich hierfür die für Amidierungen üblichen organischen Lösemittel. Hierzu gehören bevorzugt Ether wie Diethylether, Dioxan oder Tetrahydrofuran, oder chlorierte Kohlenwasserstoffe wie Methylenchlorid oder Chloroform, oder Dimethylformamid. Besonders bevorzugt wird jedoch das entsprechende Amin (III) im Überschuß, gegebenenfalls mit Tetrahydrofuran oder Dioxan als Lösemittel eingesetzt.

Die Umsetzung wird im allgemeinen in einem Temperaturbereich von 0°C bis 100°C, bevorzugt von +20°C bis +80°C durchgeführt.

Die Umsetzung erfolgt im allemeinen bei Normaldruck, es ist jedoch auch möglich, bei Unterdruck oder Überdruck zu arbeiten.

Es hat sich bei der Umsetzung als vorteilhaft erwiesen, entweder das Amin direkt als Lösemittel in sehr großem Überschuß einzusetzen, oder aber bei Verwendung eines weiteren Lösemittels in einem bis zu 10-fachen Überschuß zu verwenden.

Die Hydrolyse der diastereomeren Amide (IV) erfolgt nach üblichen Methoden, beispielsweise durch Behandeln der Amide mit Basen oder Säuren in inerten Lösemitteln.

Als inerte Lösemittel eignen sich hierfür Wasser und/oder organische Lösemittel. Als organische Lösemittel seien bevorzugt Alkohole wie Methanol, Ethanol, Propanol oder Isopropanol, oder Ether wie Diethylether, Dioxan oder Tetrahydrofuran zu nennen. Besonders bevorzugtsind Wasser sowie Wasser/Alkohol-Gemische.

Als Säuren eignen sich zur Hydrolyse der Amide die üblichen anorganischen oder organischen Säuren. Bevorzugt werden hier Salzsäure, Bromwasserstoffsäure, Schwefelsäure sowie Methansulfonsäure oder Toluolsulfonsäure verwendet

Als Basen eignen sich zur Hydrolyse der Amide die üblichen anorganischen Basen wie Natrium- oder Kaliumhydroxid oder Natrium- oder Kaliummethanolat oder -ethanolat oder Natrium- oder Kaliumcarbonat.

Bevorzugt wird die Hydrolyse der Amide im Fall der Phenethylamide in ethanolischer Salzsäure und im Fall der Phenylglycinolamide mit Natronlauge, gegebenenfalls im Beisein von Alkohol durchgeführt.

Die Hydrolyse der diastereomeren Amide (IV) erfolgt im allgemeinen in einem Temperaturbereich von 0°C bis 150°C, bevorzugt von +20°C bis +100°C.

Die Hydrolyse der Amide erfolgt im allgemeinen bei Normaldruck, kann aber auch bei Über- oder Unterdruck durchgeführt werden.

Darüberhinaus ist es auch möglich, die enantiomeren reinen Salze der Formel (I) herzustellen, indem man die entsprechenden Racemate nach üblichen Methoden der Chromatographie trennt.

Die als Ausgangsstoffe eingesetzten Amine (III) sind bekannt oder können nach an sich bekannten Methoden hergestellt werden. Bevorzugt werden erfindungsgemäß Amine der Formel (III) eingesetzt, in denen R³ für Wasserstoff und R² für Methyl oder Hydroxymethyl steht.

Die diastereomeren Amine (IV) sind neu. Sie sind wertvolle Zwischenprodukte zur Herstellung der enantiomerenreinen substituierten Pyridyl-dihydroxy-heptensäure und deren Salze.

Die erfindungsgemäße, substituierte Pyridyl-dihydroxy-heptensäure, deren Salze sowie isomeren Formen besitzen wertvolle, im Vergleich zum Stand der Technik überlegene, pharmakologische Eigenschaften, insbesondere sind sie hochwirksame Inhibitoren der 3-Hydroxy-3-methyl-glutaryl Coenzym A (HGM-CoA) Reduktase und infolge dessen Inhibitoren der Cholesterolbiosynthese. Sie können deshalb zur Behandlung von Hyperlipoproteinämie oder Arteriosklerose eingesetzt werden. Die erfindungsgemäßen Wirkstoffe bewirken außerdem eine Senkung des Cholesteringehaltes im Blut.

Die pharmakologische Wirkung der erfindungsgemäßen Stoffe wurde in folgenden Tests bestimmt:
A) Die Enzymaktivitätsbestimmung wurde modifiziert nach G.C. Ness et al., Archives of Biochemistry and Biophysics 197, 493-499 (1979) durchgeführt. Männliche Ricoratten (Körpergewicht 300 bis 400 g) wurden 11 Tage mit Altrominpulverfutter, dem 40 g Cholestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m KₓH_{y} Phosphat (Mischung aus K₂HPO₄ und KH₂PO₄ mit pH 7,2), 0,03 m Ethylendiamintetraessigsäure, 0,002 m Dithiothreit (SPE)-Puffer (Saccharose-Phosphat-Ethylendiamintetraessigsäure-Puffer) pH 7,2, homogenisiert. Anschließend wurde 15 Minuten zentrifugiert und das Sediment verworfen. Der Überstand wurde 75 Minuten bei 100 000 g sedimentiert Das Pellet wird in 1/4 Volumen SPE-Puffer aufgenommen, nochmals homogenisiert und anschließend erneut 60 Minuten zentrifugiert. Das Pellet wird mit der 5-fachen Menge ihres Volumens SPE-Puffer aufgenommen, homogenisiert und bei -78°C eingefroren und gelagert (Enzymlösung).
   Zur Testung wurden die Testverbindungen (bzw. Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37°C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP (β-Nicotinamid-adenin-dinucleotid-phosphat), 1 Einheit Glucose-6-Phosphatdehydrogenase, 35 µMol KₓH_{y}-Phosphat pH 7,2, 20 µl Enzympräparation und 56 nMol 3-Hydroxy-3-methylglutaryl Coenzym A (Glutaryl-3-¹⁴C) 100 000 dpm.
   Man inkubierte 60 Minuten bei 37°C und stoppte die Reaktion durch Zusatz von 300 µl 0,25 m HCl ab. Nach einer Nachinkubation von 60 Minuten bei 37°C wurde der Ansatz zentrifugiert und 600 µl des Überstandes auf eine 0,7 x 4 cm mit 5-Chlorid Anionenaustauscher mit einer Korngröße von 100 bis 200 mesh gefüllte Säule aufgetragen. Es wurde mit 2 ml dest. Wasser nachgewaschen und Durchlauf plus Waschwasser mit 3 mi einer Scintillationsflüssigkeit versetzt und im Scintillationszähler gezählt. IC₅₀-Werte wurden durch Auftrag der prozentualen Hemmung gegen die Konzentration der Verbindung im Test durch Intrapolation bestimmt. Zur Bestimmung der relativen inhibitorischen Potenz wurde der IC₅₀-Wert der Referenzsubstanz Mevinolin als 100 gesetzt und mit dem simultan bestimmten IC₅₀-Wert der Testverbindung verglichen.
B) Die Serum-Colesterin-senkende Wirkung der erfindungsgemäßen Verbindungen auf die Blutcholesterinwerte von Hunden wurde in mehrwöchigen Füttervngsexperimenten gefunden. Dazu wurde die zu untersuchende Substanz über einen mehrwöchigen Zeitraum einmal täglich in einer Kapsel gesunden Beagle Hunden zusammen mit dem Futter p.o. gegeben. Dem Futter war außerdem während der gesamten Versuchsperiode, d.h. vor, während und nach der Applikationsperiode der zu untersuchenden Substanz Colestyramin (4 g/100 g Futter 9 als Gallensäuresequestrant) beigemischt.
   Zweimal wöchentlich wurde den Hunden vernöses Blut abgenommen und das Serumcholesterin mit einem handelsüblichen Testkit enzymatisch bestimmt. Die Serumcholesterinwerte während der Applikationsperiode wurden mit den Serumcholesterinwerten vor der Applikationsperiode (Kontrollen) verglichen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,1 µg/kg bis etwa 100 µg/kg, bevorzugt in Gesamtmengen von etwa 1 µg/kg bis 50 µg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und dem Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Ausführungsbeispiele

### Beispiel 1

3R,5S-(+)-Natrium-erythro-(E)-7[4-(4-fluorphenyl)-2,6-diisopropyl-5-methoxymethyl-pyrid-3-yl]-3,5-dihydroxy-hept-6-enoat 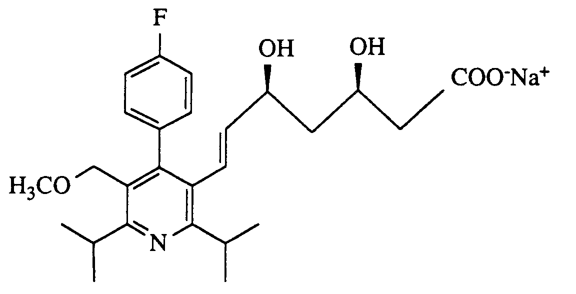 und

### Beispiel 2

3S,5R-(-)-Natrium-erythro-(E)-7-[4-(4-fluorphenyl)-2,6-diisopropyl-5-methoxymethyl-pyrid-3-yl]-3,5-dihydroxy-hept-6-enoat

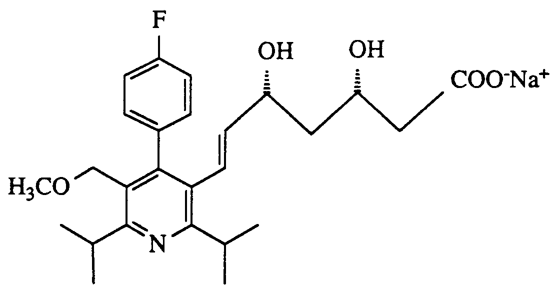

### Verfahrensvariante A - Racemat-Trennung mit R-(+)-Phenylethylamin

a) Herstellung und Trennung der diastereomeren Phenethylamide 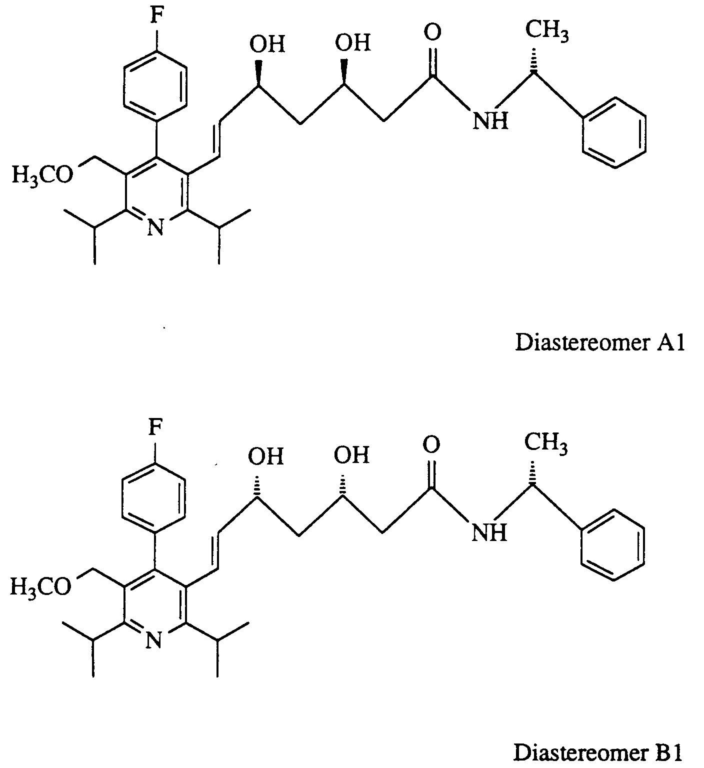
   4,7 g (10 mmol) Methyl-erythro-(E)-4-(4-fluorphenyl)-2,6-diisopropyl-5-methoxymethylpyrid-3-yl]-3,5-dthydroxy-hept-6-enoat werden in 20 ml R-(+)-Phenethylamin gelöst und 72 h auf 40°C erhitzt. Die Reaktionslösung wird auf 150 ml Wasser gegossen und die Lösung mit 1 N Salzsäure auf pH 4 gestellt. Dann wird mehrmals mit Ether extrahiert. Die vereinigten organischen Extrakte werden mit gesättigter Natriumchloridlösung gewaschen, über Magnesiumsulfat getrocknet und eingeengt. Nach einer Vorreinigung über Kieselgel 63-200 µ (Laufmittel Essigester/Petrolether 4/6 → 6/4) wird über eine 15 µ-Fertigsäule getrennt (Laufmittel Essigester/Petrolether 1/1).
   - Ausbeute:: 2,1 g Diastereomer A1 (37,4 % d.Th.),
   1,5 g Diastereomer B1 (26,6 % d.Th.).
b) Herstellung der enantiomerenreinen Natriumsalze (Bsp. 1/2)
   2,1 g (3,7 mmol) des Diastereomeren A1 werden in 70 ml 15 %igem Ethanol gelöst und nach Zugabe von 13 ml 1 n Salzsäure 48 h unter Rückfluß erhitzt. Nach dem Abkühlen wird die überstehende Lösung abfiltriert und der Rückstand mehrmals mit Ethanol verrührt. Die vereinigten Ethanol-Lösungen werden eingeengt und der Rückstand wird in 50 ml Wasser und 50 ml Dichlormethan aufgenommen. Mit 1 n Salzsäure wird der pH der Lösung auf 3,5 gestellt und die Lösung dann mehrmals mit Dichlormethan extrahiert. Die vereinigten organischen Lösungen werden über Natriumsulfat getrocknet und eingeengt. Der Rückstand wird in 50 ml Tetrahydrofuran/Wasser 1/1 aufgenommen und mit 1 n Natronlauge wird der pH der Lösung auf 7,5 gestellt. Das Tetrahydrofuran wird am Rotationsverdampfer verdampft und die zurückbleibende waßrige Losung wird lyophilisiert. Das rohe Lyophilisat wird über RP 18 gereinigt (Laufmittel: Acetonitril/Wasser 30/70). Nach Gefriertrocknung der Produktfraktionen erhält man 850 mg (48 % d.Th.) des (+)-enantiomeren Natriumsalzes (Bsp.1).
   ¹H-NMR (DMSO-d₆): δ (ppm) = 1,0 (m, 1H); 1,23 (d, 6H); 1,28 (d, 6H); 1,3 (m, 1H); 1,75 (dd, 1H); 1,98 (dd, 1H); 3,07 (s, 3H); 3,2-3,4 (m, 3H); 3,52 (m, 1H); 4,02 (m, 2H); 5,28 (dd, 1H); 6,17 (d, 1H); 7,1-7,3 (m,4H).
   Spezifische Drehung (EtOH): [α]_{D}²⁰=24,1° (c=1,0).
   Aus 1,5 g (2,6 mmol) des Diastereomeren B1 werden wie oben beschrieben, 800 mg (61,5 % d.Th.) des (-)-enantiomeren Natriumsalzes (Bsp.2) erhalten.
   Spezifische Drehung (EtOH): [α]_{D}²⁰=-23,2° (c=1,0).

### Verfahrensvariante B -Racemattrennung mit S-(+)-Phenylglycinol

a) Herstellung der diastereomeren Phenylglycinolamide 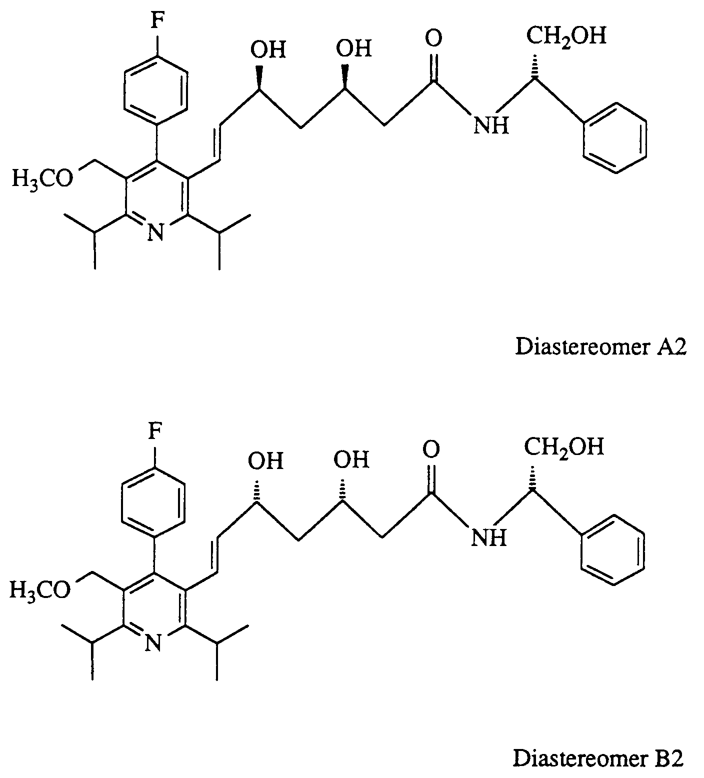
   418 g (0,88 Mol) Methyl-erythro-(E)-7-[4-(4-fluorphenyl)-2,6-diisopropyl-5-methoxymethyl-pyrid-3-yl]-3,5-dihydroxy-hept-6-enoat und 360 g (2,6 Mol) S-(+)-Phenylglycinol werden in 1 l absol. Tetrahydrofuran gelöst und 96 h auf 50°C erhitzt. Nach Abkühlen auf Raumtemperatur wird 1 l Wasser zugegeben, die Losung mit 5 n Salzsäure auf pH 4 eingestellt und 3 mal mit je 400 ml Ether extrahiert. Die vereinigten organischen Phasen werden mit 400 ml gesättigter Natriumchloridlösung gewaschen, über Natriumsulfat getrocknet und am Rotationsverdampfer eingeengt. Der Rückstand (500 g Rohprodukt) wird in zwei Portionen über eine Säule (jeweils ca. 1,8 kg Kieselgel) vorgetrennt (Laufmittel Essigester/Petrolether 8/2). Man erhält so 350 g vorgereinigtes Rohprodukt, welches fast ausschließlich aus den beiden diastereoisomeren Amiden besteht. Das vorgereinigte Rohprodukt wird in 7 Portionen à 50 g über eine Kieselgelsäule (Büchi-Säule, Lange 63 cm, ⌀ 7 cm, Kieselgel 20 µ, Probenaufgabe über 100 ml Probenschlieife) getrennt.
   Ausbeute: 195 g (38,2 % d.Th.) des Diastereomeren A2. Das Diastereomere B2 wurde nicht rein isoliert, jedoch beim Spülen der Säulen für eine eventuelle spätere Verwendung als Rohprodukt zurückgewonnen.
b) Herstellung der enantiomerenreinen Natriumsalze (Bsp.1/2)
   195 g (0,34 Mol) des diastereomerenreinen Amids A2 werden in 1 l Ethanol p.A. gelöst und nach Zugabe von 1,2 l 1 n Natronlauge wird über Nacht unter Rückfluß erhitzt. Nach Abkühlen auf Raumtemperatur wird die überstehende Lösung abdekantiert und der ölige Rückstand 3 mal mit je 50 ml Ethanol p.A. verrührt. Die Losungen werden vereinigt und eingeengt. Der Rückstand wird in 500 ml Wasser und 500 ml Methylenchlorid aufgenommen und die Lösung mit 1 n Salzsäure auf pH 3,5 eingestellt. Dann wird die organische Phase abgetrennt und die wäßrige Phase 3 mal mit je 400 ml Methylenchlorid extrahiert. Die vereinigten organischen Phasen werden getrocknet (Na₂SO₄) und eingeengt. Der Rückstand wird in 100 ml Tetrahydrofüran gelöst und die Lösung mit 500 ml Wasser verdünnt. Dann wird mit 1 n Natronlauge auf pH 7,5 gestellt, das Tetrahydrofüran am Rotationsverdampferabgezogen und die zurückbleibende wäßrige Lösung lyophilisiert.
   Man erhält 142 g rohes Lyophilisat welches zur Entsalzung in 27 Portionen à 5 g und 2 Portionen à 3,5 g über eine RP 18 Säule (Lange 40 cm, ⌀ 3 cm, Kieselgel RP 18,30 µ, Laufmittel Acetonitril/Wasser 30/70) weiter gereinigt und entsalzt wird. Alle Produktfraktionen werden vereinigt, das Acetonitril am Rotationsverdampfer abgezogen und der wäßrige Rückstand lyophilisiert.
   Ausbeute: 102 g (62,5 % d.Th.) des (+)-enantiomeren Natriumsalzes (Bsp.1).

## Patentansprüche (Patentansprüche für folgende(n) Vertragsstaat(en): AT, BE, CH, DE, DK, FR, GB, GR, IT, LI, LU, NL, SE)

1. Substituierte Pyridyl-dihydroxy-heptensäure der Formel 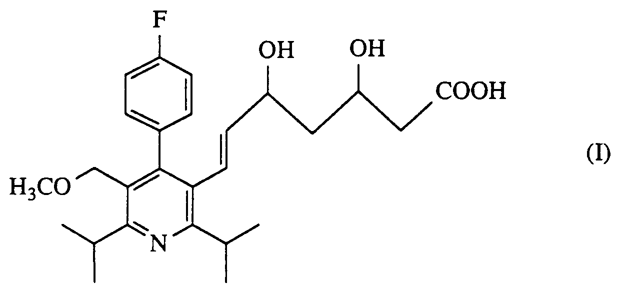 und deren Salze, gegebenenfalls in einer isomeren Form.

2. Substituierte Pyridyl-dihydroxy-heptensäure der Formel (I) nach Anspruch 1 und deren Salze in der Erythro-Konfiguration.

3. (+)-Enantiomere der substituierten Pyridyl-dihydroxy-heptensäure der Formel (I) nach Anspruch 1 und deren Salze.

4. (+)-Enantiomere substituierte Pyridyl-dihydroxy-heptensäure der Formel (I) nach Anspruch 1 und deren Salze in der Erythro-Konfiguration.

5. Natrium-, Kalium-, Magnesium- und Ammoniumsalze der substituierten Pyridyl-dihydroxy-heptensäure der Formel (I) nach Anspruch 1, gegebenenfalls in einer isomeren Form.

6. 3R,5S-(+)-Natrium-erythro-(E)-7-[4-(4-fluorphenyl)-2,6-diisopropyl-5-methoxymethyl-pyrid-3-yl]-3,5-dihydroxy-hept-6-enoat.

7. Substituierte Pyridyl-dihydroxy-heptensäuren nach den Ansprüchen 1 bis 6 zur Behandlung von Krankheiten.

8. Verfahren zur Herstellung von substituierter Pyridyl-dihydroxy-heptensäure der Formel 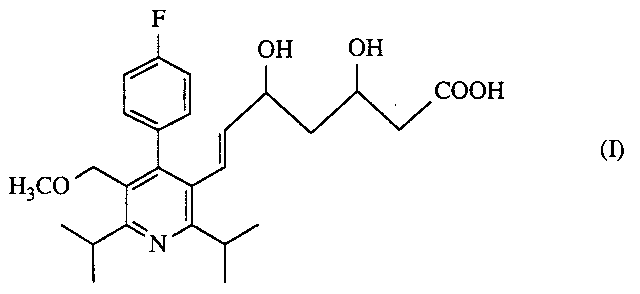 und deren Salze, gegebenenfalls in einer isomeren Form,
dadurch gekennzeichnet, daß man
[A] im Fall der racemischen Produkte die entsprechenden racemischen Ester der Formel (II) 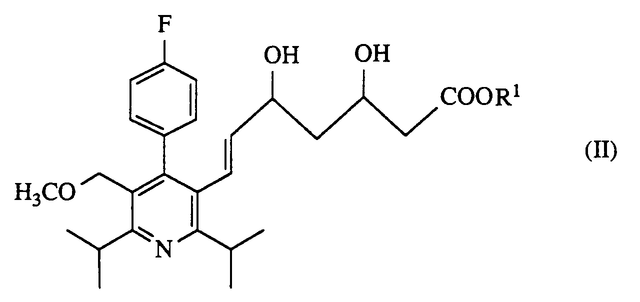 in welcher
R¹ - für C₁-C₄-Alkyl oder Benzyl steht,
hydrolysiert, oder
[B] im Fall der stereoisomer einheitlichen Produkte
zunächst die racemischen Ester der Formel (II)
mitdem (+)- oder(-)-enantiomeren Amin der Formel (III) 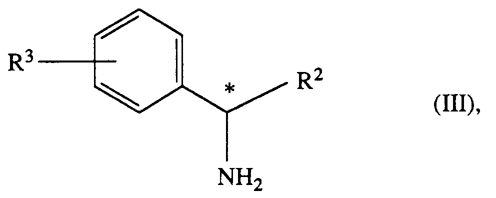 in welcher
R² - für gegebenenfalls durch Hydroxy substituiertes C₁-C₄-Alkyl steht, und
R³ - für Wasserstoff, Halogen, C₁-C₄-Alkyl oder C₁-C₄-Alkoxy steht,
in die entsprechenden diastereomeren Amide der Formel (IV) 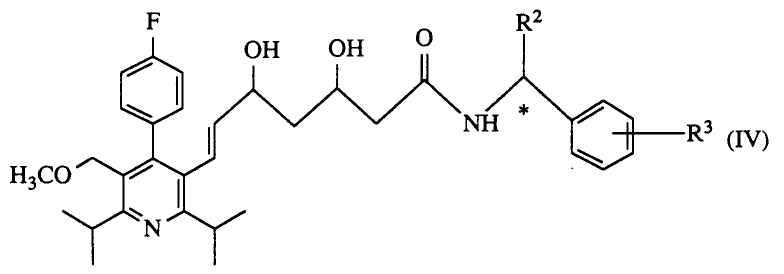 überführt,
die diastereomeren Amide durch Chromatographie oder Kristallisation in die einzelnen Diastereomeren trennt,
und anschließend die reinen diastereomeren Amide zu den enantiomerenreinen Produkten hydrolysiert.

9. Arzneimittel enthaltend substituierte Pyridyl-dihydroxy-heptensäuren nach den Ansprüchen 1 bis 6.

10. Verfahren zur Herstellung von Arzneimitteln nach Anspruch 9, dadurch gekennzeichnet, daß man die substituierte Pyridyl-dihydroxy-heptensäure nach den Ansprüchen 1 bis 6 gegebenenfalls mit Hilfe von üblichen Hilfsstoffen in eine geeignete Applikationsform überführt.

11. Verwendung der substituierten Pyridyl-dihydroxy-heptensäuren nach den Ansprüchen 1 bis 6 zur Herstellung von Arzneimitteln.

12. Substituierte Pyridyl-dihydroxy-heptensäuren nach den Ansprüchen 1 bis 6 zur Bekämpfung von Krankheiten.

13. Diastereomere Amide der Formel 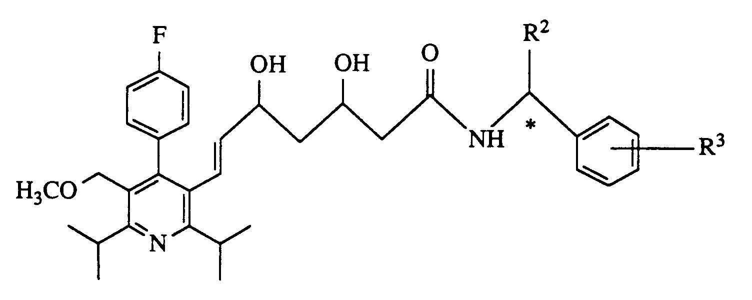 in welcher
R² - für gegebenenfalls durch Hydroxy substituiertes C₁-C₄-Alkyl steht,
und
R³ für Wasserstoff, C₁-C₄-Alkyl, Halogen oder C₁-C₄-Alkoxy steht.

## Patentansprüche (Patentansprüche für folgende(n) Vertragsstaat(en): ES)

1. Verfahren zur Herstellung von substituierten Pyridyl-dihydroxy-heptensäure der Formel 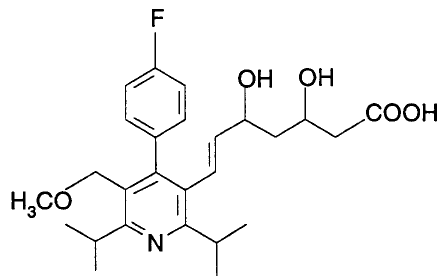 und deren Salze, gegebenenfalls in einer isomeren Form, dadurch gekennzeichnet, daß man
[A] im Fall der racemischen Produkte die entsprechenden racemischen Ester der Formel (II) 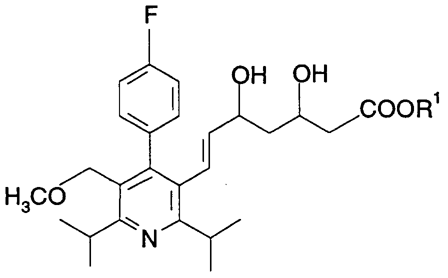 in welcher
R¹ für C₁-C₄-Alkyl oder Benzyl steht, hydrolysiert, oder
[B] im Fall der stereoisomer einheitlichen Produkte zunächst die racemischen Ester der Formel (II) mit dem (+)- oder (-)-enantiomeren Amin der Formel (III) 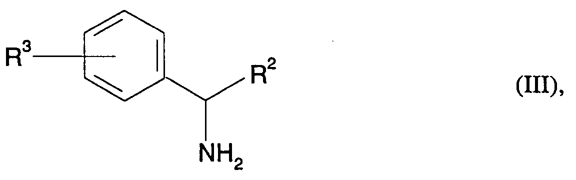 in welcher
R² für gegebenenfalls durch Hydroxy substituiertes C₁-C₄-Alkyl steht,
und
R³ für Wasserstoff, Halogen, C₁-C₄-Alkoxy steht,
in die entsprechenden diastereomeren Amide der Formel (IV) 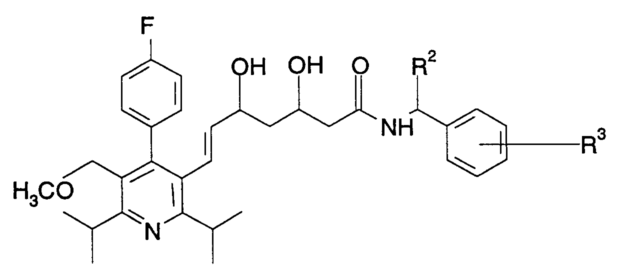
die diastereomeren Amide durch Chromatographie oder Kristallisation in die einzelnen Diastereomeren trennt, und anschließend die reinen diastereomeren Amide zu den enantiomerenreinen Produkten hydrolysiert.

2. Verfahren nach Anspruch 1 zur Herstellung von Verbindungen der Formel (I) in der Erythroform.

3. Verfahren nach Anspruch 1 zur Herstellung der (+)-Enantiomeren der Formel (I).

4. Verfahren nach Anspruch 1 zur Herstellung der (+)-Enantiomeren in der Erythroform.

5. Verfahren nach Anspruch 1 zur Herstellung der Natrium-, Kalium-, Magnesium- und Ammoniumsalze.

6. Verfahren nach Anspruch 1 zur Herstellung von 3R,5S-(+)-Natrium-erythro-(E)-7-[4-(4-fluorphenyl)-2,6-diisopropyl-5-methoxymethyl-pyrid-3-yl]-3,5-dihydroxy-hept-6-enoat.

## Claims (Claims for the following Contracting State(s): AT, BE, CH, DE, DK, FR, GB, GR, IT, LI, LU, NL, SE)

1. Substituted pyridyl-dihydroxy-heptenoic acid of the formula 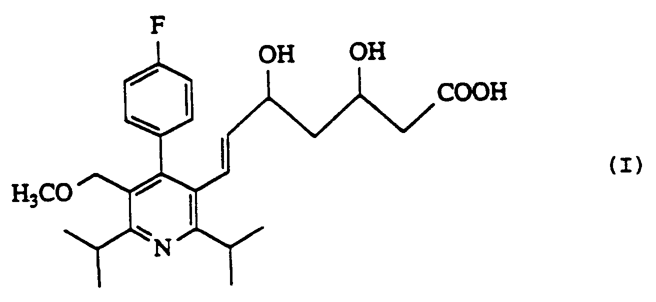 and its salts, if desired in an isomeric form.

2. Substituted pyridyl-dihydroxy-heptenoic acid of the formula (I) according to Claim 1 and its salts in the erythro configuration.

3. (+)-Enantiomers of the substituted pyridyl-dihydroxy-heptenoic acid of the formula (I) according to Claim 1 and its salts.

4. (+)-Enantiomers of substituted pyridyl-dihydroxy-heptenoic acid of the formula (I) according to Claim 1 and its salts in the erythro configuration.

5. Sodium, potassium, magnesium and ammonium salts of the substituted pyridyl-dihydroxy-heptenoic acid of the formula (I) according to Claim 1, if desired in an isomeric form.

6. Sodium 3R,5S-(+)-erythro-(E)-7-[4-(4-fluorophenyl)-2,6-diisopropyl-5-methoxymethyl-pyrid-3-yl]-3,5-dihydroxy-hept-6-enoate.

7. Substituted pyridyl-dihydroxy-heptenoic acids according to Claims 1 to 6 for the treatment of diseases.

8. Process for the preparation of substituted pyridyl-dihydroxy-heptenoic acid of the formula 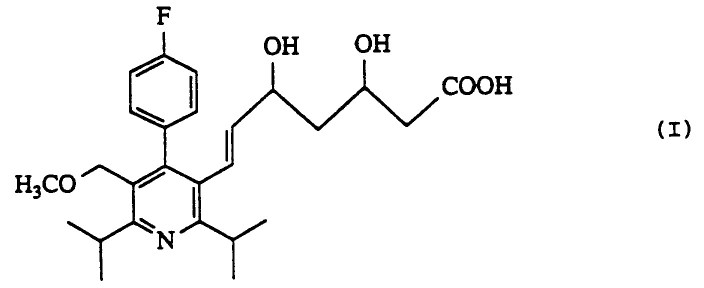 and its salts, if desired in an isomeric form,
characterised in that
[A] in the case of the racemic products, the corresponding racemic esters of the formula (II) 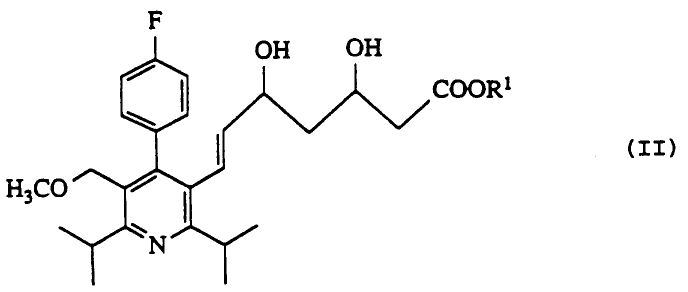 in which
R¹- represents C₁-C₄-alkyl or benzyl,
are hydrolysed, or
[B] in the case of the stereoisomerically homogeneous products
first the racemic esters of the formula (II) are converted
using the (+)- or (-)-enantiomeric amine of the formula (III) 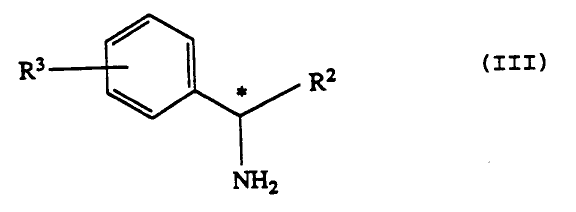 in which
R²- represents C₁-C₄-alkyl which is optionally substituted by hydroxyl and
R³- represents hydrogen, halogen, C₁-C₄-alkyl or C₁-C₄-alkoxy,
into the corresponding diastereomeric amides of the formula (IV) 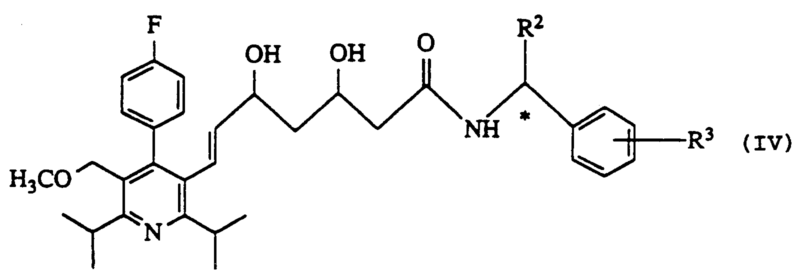
the diastereomeric amides are separated into the individual diastereomers by chromatography or crystallisation,
and then the pure diastereomeric amides are hydrolysed to give the enantiomerically pure products.

9. Medicaments containing substituted pyridyl-dihydroxy-heptenoic acids according to Claims 1 to 6.

10. Process for the production of medicaments according to Claim 9, characterised in that the substituted pyridyl-dihydroxy-heptenoic acid according to Claims 1 to 6 is converted into a suitable administration form, if appropriate with the aid of customary auxiliaries.

11. Use of the substituted pyridyl-dihydroxy-heptenoic acids according to Claims 1 to 6 for the production of medicaments.

12. Substituted pyridyl-dihydroxy-heptenoic acids according to Claims 1 to 6 for the control of diseases.

13. Diastereomeric amides of the formula 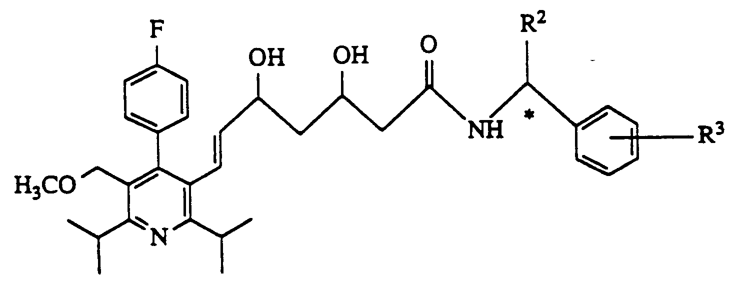 in which
R²- represents C₁-C₄-alkyl which is optionally substituted by hydroxyl,
and
R³ represents hydrogen, C₁-C₄-alkyl, halogen or C₁-C₄-alkoxy.

## Claims (Claims for the following Contracting State(s): ES)

1. Process for the preparation of substituted pyridyl-dihydroxy-heptenoic acid of the formula 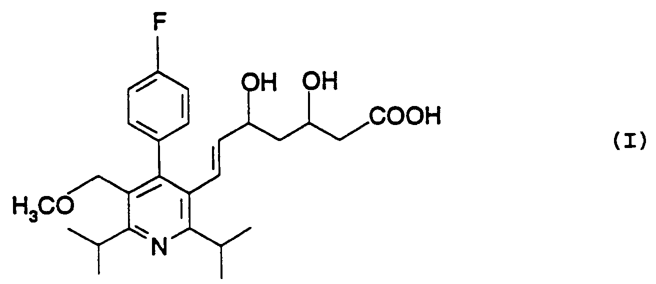 and its salts, if desired in an isomeric form,
characterised in that
[A] in the case of the racemic products, the corresponding racemic esters of the formula (II) 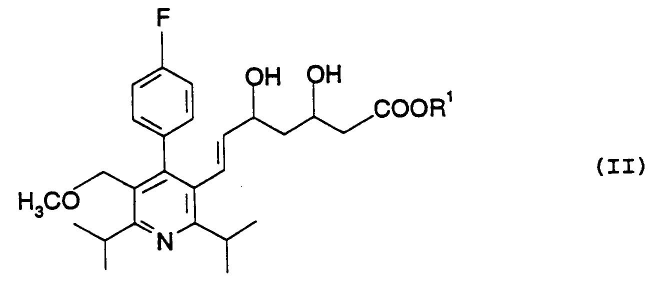 in which
R¹ represents C₁-C₄-alkyl or benzyl,
are hydrolysed, or
[B] in the case of the stereoisomerically homogeneous products first the racemic esters of the formula (II) are converted using the (+)- or (-)-enantiomeric amine of the formula (III) 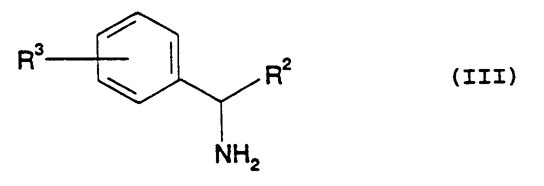 in which
R² represents C₁-C₄-alkyl which is optionally substituted by hydroxyl and
R³ represents hydrogen, halogen or C₁-C₄-alkoxy,
into the corresponding diastereomeric amides of the formula (IV) 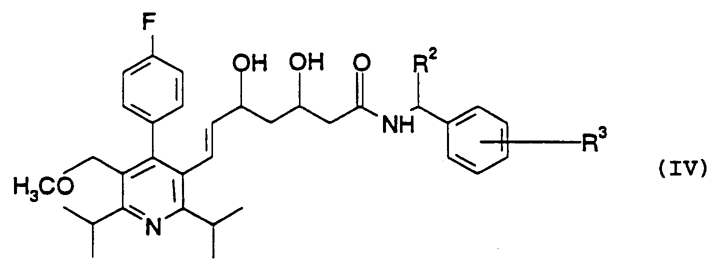
the diastereomeric amides are separated into the individual diastereomers by chromatography or crystallisation, and then the pure diastereomeric amides are hydrolysed to give the enantiomerically pure products.

2. Process according to Claim 1 for preparing compounds of the formula (I) in the erythro configuration.

3. Process according to Claim 1 for preparing the (+)-enantiomers of the formula (I).

4. Process according to Claim 1 for preparing the (+)-enantiomers in the erythro configuration.

5. Process according to Claim 1 for preparing the sodium, potassium, magnesium and ammonium salts.

6. Process according to Claim 1 for preparing sodium 3R,5S-(+)-erythro-(E)-7-[4-(4-fluorophenyl)-2,6-diisopropyl-5-methoxymethyl-pyrid-3-yl]-3,5-dihydroxy-hept-6-enoate.

## Revendications (Revendications pour l'(les) Etat(s) contractant(s) suivant(s): AT, BE, CH, DE, DK, FR, GB, GR, IT, LI, LU, NL, SE)

1. Acide pyridyl-dihydroxy-hepténoïque substitué répondant à la formule 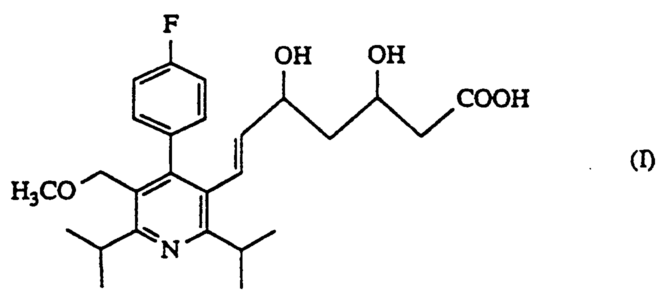 et ses sels, éventuellement dans une forme isomère.

2. Acide pyridyl-dihydroxy-hepténoïque substitué de formule (I) selon la revendication 1 et ses sels dans la configuration érythro.

3. Enantiomères (+) de l'acide pyridyl-dihydroxy-hepténoïque substitué de formule (I) selon la revendication 1 et leurs sels.

4. Acide énantiomère (+) pyridyl-dihydroxy-hepténoïque substitué de formule (I) selon la revendication 1 et ses sels dans la configuration érythro.

5. Sel de sodium, de potassium, de magnésium et d'ammonium de l'acide pyridyl-dihydroxy-hepténoïque substitué de formule (I) selon la revendication 1, éventuellement dans une forme isomère.

6. 3R,5S-(+)-érythro-(E)-7-[4-(4-fluorophényl)-2,6-diisopropyl-5-méthoxyméthyl-pyrid-3-yl]-3,5-dihydroxy-hept-6-énoate de sodium.

7. Acides pyridyl-dihydroxy-hepténoïques substitués selon les revendications 1 à 6, pour le traitement de maladies.

8. Procédé pour la préparation de l'acide pyridyl-dihydroxy-hepténoïque substitué de formule 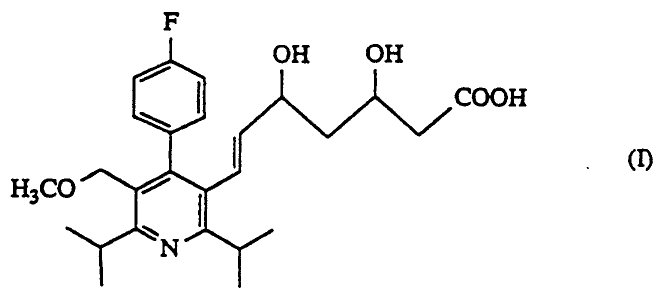 et de ses sels éventuellement dans une forme isomère, caractérisé en ce que
[A] dans le cas des produits racémiques, on hydrolyse les esters racémiques correspondant de formule (II) 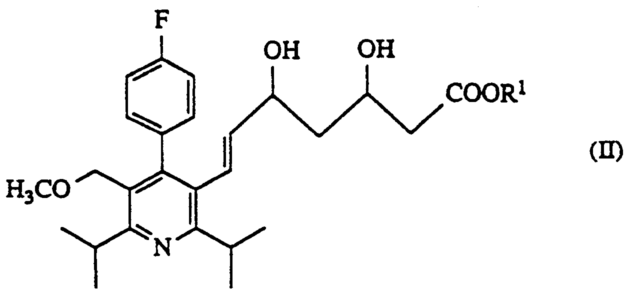 dans laquelle
R¹ représente un groupe alkyle en C₁-C₄ ou un groupe benzyle,
ou bien
[B] dans le cas des produits homogènes quant à la stéréo-isomérie,
on transforme d'abord les esters racémiques de formule (II)
avec l'amine énantiomère (+) ou (-) de formule (III) 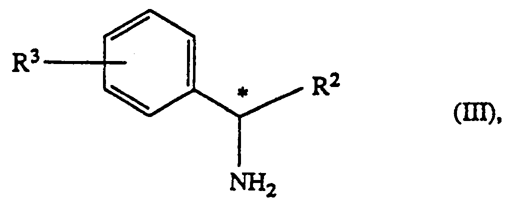 dans laquelle
R² représente un groupe alkyle en C₁-C₄ éventuellement substitué par un groupe hydroxyle, et
R³ représente un atome d'hydrogène, un atome d'halogène, un groupe alkyle en C₁-C₄ ou un groupe alcoxy en C₁-C₄,
en amides diastéréo-isomères correspondants de formule (IV) 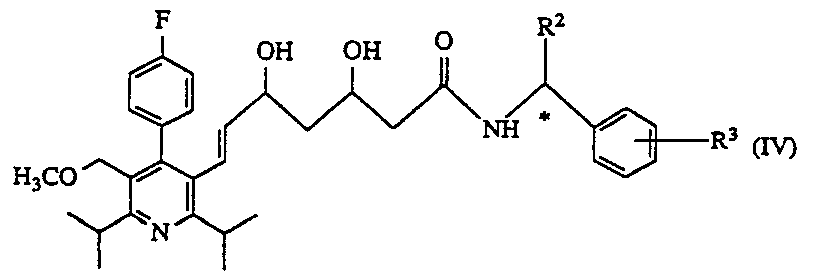
on dédouble les amides diastéréo-isomères par chromatographie ou par cristallisation en diastéréo-isomères individuels,
et ensuite, on hydrolyse les formes pures des amides diastéréo-isomères pour obtenir la forme pure des énantiomères du produit.

9. Médicament contenant des acides pyridyl-dihydroxy-hepténoïques substitués selon les revendications 1 à 6.

10. Procédé pour la préparation de médicaments selon la revendication 9, caractérisé en ce qu'on transforme l'acide pyridyl-dihydroxy-hepténoïque substitué selon les revendications 1 à 6, éventuellement à l'aide d'adjuvants habituels, dans une forme d'application appropriée.

11. Utilisation des acides pyridyl-dihydroxy-hepténoïques substitués selon les revendications 1 à 6, pour la préparation de médicaments.

12. Acides pyridyl-dihydroxy-hepténoïques substitués selon les revendications 1 à 6 pour lutter contre des maladies.

13. Amides diastéréo-isomères de formule 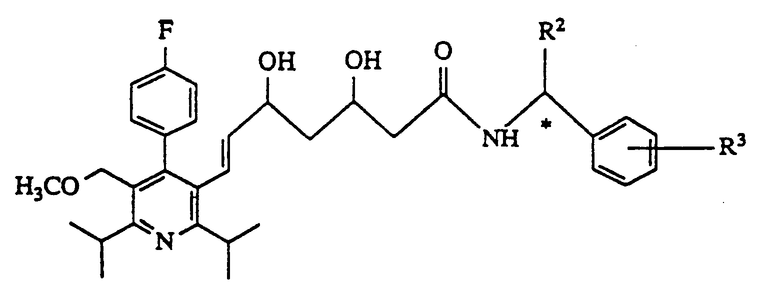 dans laquelle
R² représente un groupe alkyle en C₁-C₄ éventuellement substitué par un groupe hydroxyle, et
R³ représente un atome d'hydrogène, un groupe alkyle en C₁-C₄, un atome d'halogène ou un groupe alcoxy en C₁-C₄.

## Revendications (Revendications pour l'(les) Etat(s) contractant(s) suivant(s): ES)

1. Procédé pour la préparation de l'acide pyridyl-dihydroxy-hepténoïque substitué de formule 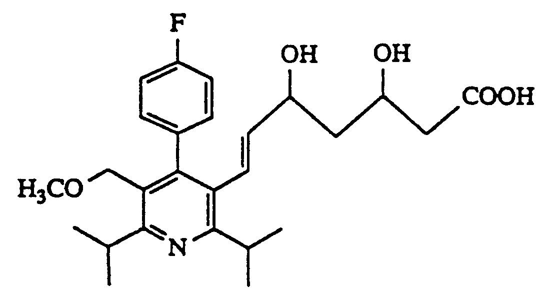 et de ses sels éventuellement dans une forme isomère, caractérisé en ce que
[A] dans le cas des produits racémiques, on hydrolyse les esters racémiques correspondant de formule (II) 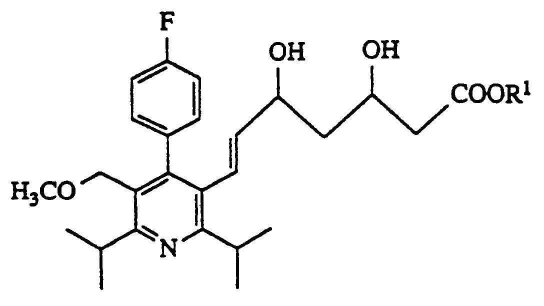 dans laquelle
R¹ représente un groupe alkyle en C₁-C₄ ou un groupe benzyle,
ou bien
[B] dans le cas des produits homogènes quant à la stéréo-isomérie,
on transforme d'abord les esters racémiques de formule (II)
avec l'amine énantiomère (+) ou (-) de formule (III) 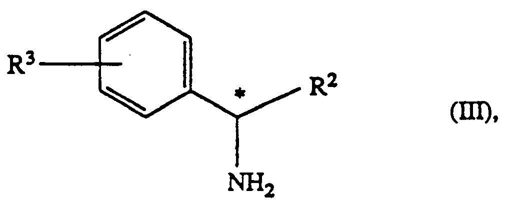 dans laquelle
R² représente un groupe alkyle en C₁-C₄ éventuellement substitué par un groupe hydroxyle, et
R³ représente un atome d'hydrogène, un atome d'halogène, un groupe alcoxy en C₁-C₄,
en amides diastéréo-isomères correspondants de formule (IV) 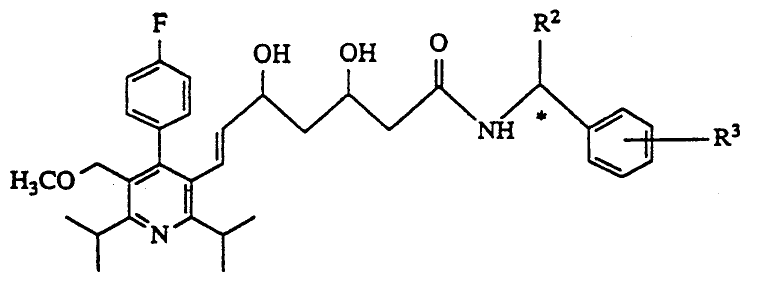
on dédouble les amides diastéréo-isomères par chromatographie ou par cristallisation en diastéréo-isomères individuels, et ensuite, on hydrolyse la forme pure des amides diastéréo-isomères pour obtenir la forme pure des énantiomères du produit.

2. Procédé selon la revendication 1 pour la préparation de composés de formule (I) dans la forme érythro.

3. Procédé selon la revendication 1 pour la préparation des énantiomères (+) de formule (I).

4. Procédé selon la revendication 1 pour la préparation des énantiomères (+) dans la forme érythro.

5. Procédé selon la revendication 1 pour la préparation des sels de sodium, de potassium, de magnésium et d'ammonium.

6. Procédé selon la revendication 1 pour la préparation du 3R,5S-(+)-érythro-(E)-7-[4-(4-fluorophényl)-2,6-diisopropyl-5-méthoxyméthyl-pyrid-3-yl]-3,5-dihydroxy-hept-6-énoate de sodium.
